(19) Europäisches Patentamt / European Patent Office / Office européen des brevets

(11) **EP 2 402 299 A1**

(12) **EUROPEAN PATENT APPLICATION**

(43) Date of publication:
**04.01.2012 Bulletin 2012/01**

(51) Int Cl.:
*C07C 1/04* (2006.01)   *C07C 11/02* (2006.01)
*B01J 23/889* (2006.01)   *B01J 29/076* (2006.01)
*B01J 29/26* (2006.01)   *B01J 37/03* (2006.01)

(21) Application number: **10167922.3**

(22) Date of filing: **30.06.2010**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(71) Applicant: **Shell Internationale Research Maatschappij B.V.**
**2596 HR The Hague (NL)**

(72) Inventor: **Verhaak, Michael Johannes Franciscus Maria**
**1031 HV Amserdam (NL)**

(54) **Process for preparing olefins from synthesis gas using a cobalt and manganese containing catalyst**

(57) The invention relates to a process for preparing olefins from synthesis gas, wherein a catalyst which contains cobalt and manganese is reduced at a temperature higher than 350 °C, and the reduced catalyst is contacted with synthesis gas to effect reaction of the synthesis gas into olefins. Further, the invention relates to a process for preparing such catalyst, and to the catalyst so obtained.

EP 2 402 299 A1

**Description**

The present invention relates to a process for preparing olefins from synthesis gas (syngas) using a catalyst which contains cobalt and manganese.

Syngas is a gas mixture which comprises hydrogen ($H_2$) and carbon monoxide (CO). Syngas may be converted into hydrocarbons by a catalytic process which is usually referred to as the Fischer-Tropsch (FT) process. Catalysts which are commonly used in the FT process, contain iron (Fe), cobalt (Co), nickel (Ni) and/or ruthenium (Ru) as the catalytically active metal. This is for example described by Van der Laan et al. in Catal. Rev.-Sci. Eng., 41, 1999, p. 255 ff.

There is a special interest for the selective and direct production of ethylene, propylene and butylene ($C_2$-$C_4$ olefins) from syngas, which are important chemical hydrocarbon feedstocks. Cobalt-manganese (Co-Mn) catalysts have been shown to have a relatively high selectivity to these olefins. Mirzaei et al. disclose in Applied Catalysis A: General, 306, 2006, p. 98 ff., the preparation of precipitated cobalt manganese catalyst and the use thereof in producing lower olefins from syngas. Said article discloses that before syngas exposure, the catalyst was reduced in a hydrogen-nitrogen stream at 350 °C. The syngas reaction was then carried out at 350-500 °C.

An object of the present invention is to find a cobalt and manganese containing catalyst which has good selectivity and/or activity in the preparation of olefins from syngas.

Surprisingly it was found that said object is achieved by reducing the cobalt and manganese containing catalyst at a temperature higher than 350 °C.

Accordingly, the present invention relates to a process for preparing olefins from synthesis gas, wherein a catalyst which contains cobalt and manganese is reduced at a temperature higher than 350 °C, and the reduced catalyst is contacted with synthesis gas to effect reaction of the synthesis gas into olefins.

Said reduction is performed by subjecting the catalyst to a gas stream comprising a reducing gas. Suitable reducing gases are hydrogen, carbon monoxide and mixtures of hydrogen and carbon monoxide. A mixture which comprises hydrogen and carbon monoxide is herein also referred to as synthesis gas or syngas. In a case where syngas is used as the reducing gas, the syngas may have a volume ratio of carbon monoxide to hydrogen of from 3:1 to 1:3. Preferably, in such case, the syngas has a volume ratio of carbon monoxide to hydrogen of from 1:0.5 to 1:2.5.

It is preferred that said reducing gas consists of hydrogen. In particular, it is preferred that before the catalyst is contacted with synthesis gas to effect reaction of the synthesis gas into olefins, the catalyst is subjected to a gas stream comprising a reducing gas consisting of hydrogen.

Said gas stream comprising a reducing gas may consist of a reducing gas, which means that it only contains a reducing gas as described above, and no other gases, such as inert gases.

Said gas stream comprising a reducing gas may additionally comprise an inert gas. Within the present specification, inert gas means a gas which does not reduce the catalyst or react in any other way. Suitable inert gases are nitrogen and argon. It is preferred that said gas stream comprising a reducing gas consists of from 1 to 99 vol.% of reducing gas, more preferably 10 to 90 vol.% of reducing gas, more preferably 20 to 80 vol.% of reducing gas, more preferably 30 to 70 vol.% of reducing gas, most preferably 40 to 60 vol.% of reducing gas, the remainder of the stream comprising an inert gas. Preferably, said reducing gas comprises hydrogen, carbon monoxide or a mixture of hydrogen and carbon monoxide, as described above. More preferably, said reducing gas consists of hydrogen.

The duration of the reduction treatment is not critical, and may be of from 1 to 24 hours, suitably of from 1 to 10 hours, more suitably of from 1 to 8 hours. The reduction treatment does not have to result in a completely reduced catalyst. For example, further reduction may take place during the reaction of the synthesis gas into olefins. Further, it is envisaged that in a case where the reducing gas comprises syngas as described above, reduction of the catalyst and reaction of the syngas into olefins may proceed simultaneously. In particular, the present process for preparing olefins from synthesis gas may consist of contacting the catalyst which contains cobalt and manganese with synthesis gas at a temperature higher than 350 °C. In the latter case, reduction of the catalyst and reaction of the syngas into olefins proceed simultaneously.

The catalyst is reduced at a temperature which is higher than 350 °C, preferably at least 375 °C, more preferably at least 400 °C, even more preferably at least 425 °C, most preferably at least 450 °C. Further, said reduction temperature is suitably lower than 800 °C. Preferably, said reduction temperature is at most 700 °C, more preferably at most 600 °C, even more preferably at most 500 °C, most preferably at most 475 °C. Said reduction temperature may be of from 200 to 700 °C, suitably 300 to 600 °C, and preferably 400 to 500 °C, more preferably 425 to 475 °C, and most preferably 435 to 465 °C.

Preferably, the olefins to be produced in the present process are light olefins, suitably unbranched $C_2$-$C_6$ alkenes, more preferably unbranched $C_2$-$C_4$ alkenes, and most preferably ethylene and propylene.

The catalyst to be used in the present process contains cobalt and manganese. Preferably, said catalyst additionally contains a third element selected from the group consisting of aluminium, gallium, indium, thallium, tin, lead and bismuth.

The atomic ratio of manganese to cobalt in the catalyst to be used in the present process may vary within wide limits. It may be of from 20:1 to 1:20, preferably 10:1 to 1:10, more preferably 10:1 to 1:2, most preferably 8:1 to 1:1.

The amount of the optional third element in the catalyst to be used in the present process may also vary within wide limits. It may be of from 0.01 to 5 wt.%, preferably 0.05 to 2.5 wt.%, more preferably 0.1 to 2 wt.%, even more preferably 0.2 to 1 wt.%, most preferably 0.3 to 0.8 wt.% based on total amount of the catalyst including any support.

Preferably, the catalyst to be used in the present invention is a supported catalyst. Examples of suitable supports for the present catalyst are titania ($TiO_2$), silica ($SiO_2$), alumina ($Al_2O_3$) and zeolite. A suitable example of such zeolite support is H-mordenite.

The catalyst to be used in the present invention may be prepared in a variety of ways. A supported catalyst may be prepared by means of a process comprising co-precipitation of a manganese salt and a cobalt salt on a support, optionally followed by impregnation with a salt of the optional third element, and finally reduction at a temperature higher than 350 °C.

Accordingly, the present invention also relates to a process for preparing a supported catalyst, suitably for use in preparing olefins from synthesis gas, wherein the catalyst contains cobalt, manganese and optionally a third element selected from the group consisting of aluminium, gallium, indium, thallium, tin, lead and bismuth, preferably tin, and is prepared by co-precipitation of a manganese salt and a cobalt salt on a support, optionally followed by impregnation with a salt of the third element, and finally reduction at a temperature higher than 350 °C. The embodiments and preferences as discussed above regarding reduction, also apply to the reduction as part of said process for preparing a supported catalyst.

Preferably, said process for preparing a supported catalyst comprises combining the support with an aqueous solution containing the manganese salt and the cobalt salt, or with an aqueous solution containing the manganese salt and another aqueous solution containing the cobalt salt, resulting in a dispersion, and contacting the dispersion with a basic solution to effect the co-precipitation, and optionally contacting the solid (precipitate) from the dispersion with an impregnating solution containing the salt of the third element, and finally reduction at a temperature higher than 350 °C.

Said manganese salt may be manganese nitrate. Said cobalt salt may be cobalt nitrate. Preferably, said solution or solutions containing the manganese salt and the cobalt salt is or are heated before combining with the support, for example at 50 to 90 °C, preferably 60 to 80 °C. The basic solution to be contacted with the dispersion may be a sodium carbonate solution. Preferably, said basic solution is heated before contacting with the dispersion, for example at 50 to 90 °C, preferably 60 to 80 °C. The amount of base in said solution should be sufficient to allow for co-precipitation of the manganese and cobalt salts on the support. This may be achieved by adding such amount of base that a pH of 8 or greater, preferably a pH of 9 or greater and most preferably a pH of 9.5 or greater is achieved in the dispersion. The solid (precipitate) in the dispersion or slurry resulting from the addition of the basic solution can be removed therefrom by filtration. The separated solid may be washed with water in order to remove substantially all base from the basic solution. Possibly after drying the separated solid, for example at a temperature in the range of from 80 to 120 °C, it may further be calcined in the presence of air, at a temperature in the range of from 200 to 800 °C, preferably 300 to 700 °C, more preferably 400 to 600 °C, most preferably 450 to 550 °C.

Optionally, the above-mentioned separated solid is then to be impregnated with the salt of the third element, for example by contacting with an impregnating solution containing the salt of the third element, preferably tin. Said salt of the third element is any soluble salt, such as a nitrate, sulfate, chloride, hydroxide or organic acid salt of said third element. Preferably, said salt of the third element is a nitrate, sulfate, chloride, hydroxide or organic acid salt of tin. Preferred organic acid salts of tin are tin tartrate and tin acetate. The paste that results from contacting the above-mentioned separated solid with an impregnating solution containing the salt of the third element may be heated for example at 40 to 80 °C, preferably 50 to 70 °C. Possibly after drying the impregnated catalyst, for example at a temperature in the range of from 80 to 120 °C, it may further be calcined in the presence of air, at a temperature in the range of from 200 to 800 °C, preferably 300 to 700 °C, more preferably 400 to 600 °C, most preferably 450 to 550 °C.

Finally, the calcined or not calcined catalyst is reduced at a temperature higher than 350 °C.

The present invention also relates to a catalyst obtainable by the catalyst preparation process(es) as described above.

The catalyst may be provided to a syngas conversion reactor as a fixed bed, a fluidized bed, or a slurry.

The synthesis gas to be used in the present process for preparing olefins from synthesis gas can be produced by generally known processes, as described for example in Weissermel et al., Industrial Organic Chemistry, Wiley-VCH, Weinheim, 2003, p. 15-24. For example, syngas can be produced by reaction of coal or methane with steam or by reaction of methane with carbon dioxide. In the present process, the syngas to be reacted into olefins may have a volume ratio of carbon monoxide to hydrogen of from 3:1 to 1:3. Preferably, a syngas is used which has a volume ratio of carbon monoxide to hydrogen of from 1:0.5 to 1:2.5.

The reaction temperature may be of from 100 to 700 °C. Preferably, the reaction temperature is at least 200 °C, more preferably at least 225 °C, more preferably at least 250 °C, more preferably at least 275 °C, most preferably at least 285 °C. Further, preferably, the reaction temperature is at most 600 °C, more preferably at most 500 °C, more preferably at most 375 °C, more preferably lower than 350 °C, more preferably at most 325 °C, most preferably at most 315 °C.

Preferably, in the present invention, the reaction temperature is equal to or lower than the reduction temperature. In particular, it is preferred that the catalyst is reduced at a temperature higher than 350 °C as described above, and the reduced catalyst is then contacted with synthesis gas at a temperature of from 100 to lower than 350 °C, preferably at most 325°C, more preferably at most 315°C, to effect reaction of the synthesis gas into olefins. In the latter case, said reaction temperature (of at least 100 °C) is preferably at least 200 °C, more preferably at least 225 °C, more preferably at least 250 °C, more preferably at least 275 °C, most preferably at least 285 °C.

The pressure in the present process may be of from 1 to 60 bar, preferably of from 10 to 50 bar. The GHSV (gas hourly space velocity) is generally from 100 to 30,000 parts by volume of feed stream per part by volume of catalyst per hour, in $1/(1*hr)$ or $hr^{-1}$.

The invention is further illustrated by the following Examples.

Examples

Catalyst preparation

A catalyst containing cobalt and manganese with H-mordenite as support, hereinafter referred to as base-case catalyst, was prepared by means of a co-precipitation procedure.

Manganese nitrate ($Mn(NO_3)_2.6H_2O$) and cobalt nitrate ($CO(NO_3)_2.6H_2O$) were dissolved in demineralized water. The amounts of cobalt nitrate and manganese nitrate were chosen such that the Mn/Co atomic ratio in the final catalyst was 6.

The resulting aqueous solution was heated to 70 °C. H-mordenite was then added to the aqueous solution while stirring to obtain a dispersion. The amount of H-mordenite was chosen such that the weight percentage of H-mordenite was 15 wt.%, on the basis of the total weight of cobalt, manganese and H-mordenite.

In a separate vessel, sodium carbonate was dissolved in demineralized water to obtain a 1.0 M (mole/l) solution which was also heated to 70 °C. Basic carbonate solution was then added to the dispersion while stirring to achieve a final pH of about 10.

The slurry obtained was left stirring for another 30 minutes and then filtered. The solid residue was washed with warm demineralized water until free of sodium ions, as determined by atomic adsorption analysis. The solids were then dried at 110 °C for 16 hours resulting in a catalyst herein referred to as Catalyst A.

A promoted catalyst was then prepared from the above base-case catalyst, containing cobalt and manganese with H-mordenite as support, by means of an impregnation procedure.

Tin tartrate (tin(II) 2,3-dihydroxybutanedioate) was dissolved in demineralized water. The amount of tin tartrate was chosen such that the weight percentage of tin was 0.5 wt.%, on the basis of the total weight of tin and base-case catalyst. The base-case catalyst was impregnated with the tin tartrate solution resulting in a thick paste. The paste was heated to 60 °C in an oven and regularly stirred to homogenize the paste and finally it was left to dry for 16 hours. The dried material was calcined at 500 °C in air (heating rate 2 °C/minute), resulting in a catalyst herein referred to as Catalyst B.

Use of catalysts in syngas conversion

The above Catalyst A (unpromoted) and Catalyst B (promoted with 0.5 wt.% of Sn) were then evaluated in the conversion of syngas in a small-scale, continuous flow, fixed bed setup. Prior to exposure to syngas, these catalysts were reduced in a flow of 50 vol.% hydrogen in nitrogen for 5 hours at a reduction temperature of 350 °C (Catalysts Al and B1 which are not in accordance with the invention) or 450 °C (Catalysts A2 and B2 which are in accordance with the invention).

The catalyst was then cooled down, in the presence of said reducing gas, to 300 °C. Then the gas stream was switched to a stream of a mixture of carbon monoxide and hydrogen diluted in nitrogen. The overall gas composition of the latter stream was 33:17:50 (in vol.%) for $CO:H_2:N_2$ ($H_2/CO$ volume ratio was 0.5). The system was pressurized to 15 bar gauge (barg). The pressure and temperature during the reaction were maintained at 15 barg and 300 °C, respectively. The gas hourly space velocity (GHSV) was 1,000 $hr^{-1}$.

The product gas stream was analyzed with a gas chromatograph (GC) equipped with a flame ionization detector (FID) and a thermal conductivity detector (TCD). Table 1 shows the CO conversion (indicated as "CO") and the selectivities to carbon dioxide (indicated as "$CO_2$") and to the various hydrocarbon products (indicated by the molecular formula of the hydrocarbon product in question) as obtained in syngas conversions using Catalysts A1, A2, B1 and B2, at about 70 hours after the introduction of the syngas stream.

CO conversion is herein defined as:

$$((\text{moles } CO_{in} - \text{moles } CO_{out})/\text{moles } CO_{in})*100\%$$

Selectivity to $CO_2$ is herein defined as:

$$(\text{moles } CO_2)/(\text{moles converted CO})*100\%$$

Selectivity to hydrocarbon product "x" is herein defined as:

$$((\text{weight product "x"})/\text{total weight of all hydrocarbon}$$

$$\text{products excluding carbon dioxide})*100\%$$

Table 1

| Catalyst | CO | $CO_2$ | $CH_4$ | $C_2H_4$ olefin | $C_2H_6$ | $C_3H_6$ olefin | $C_3H_8$ | $C_4H_8$ olefin | $C_4H_{10}$ | $C_{5+}$ | Total $C_2$-$C_4$ olefins |
|---|---|---|---|---|---|---|---|---|---|---|---|
| A1 | 49.1 | 47.8 | 13.7 | 13.5 | 2.4 | 23.5 | 0.8 | 13.8 | 0.5 | 31.8 | 50.8 |
| B1 | 25.5 | 49.9 | 13.8 | 13.1 | 1.8 | 27.4 | 1.2 | 13.6 | 0.8 | 28.3 | 54.1 |
| A2 | 48.6 | 46.9 | 13.4 | 14.1 | 2.5 | 25.9 | 0.9 | 15.3 | 0.6 | 27.3 | 55.3 |
| B2 | 21.8 | 50.5 | 11.7 | 11.0 | 1.6 | 34.0 | 1.3 | 16.9 | 0.8 | 22.7 | 61.9 |

Upon comparing the results from Table 1 between the A1 and A2 catalysts and the B1 and B2 catalysts, respectively, it appears that the total selectivity to $C_2$-$C_4$ olefins, and in particular the selectivity to propylene ($C_3H_6$), increases significantly because of the higher reduction temperature of 450 °C for the A2 and B2 catalysts.

Further, upon comparing the results from Table 1 between the A2 and B2 catalysts, which both had been reduced at the higher reduction temperature of 450 °C, it appears that the total selectivity to $C_2$-$C_4$ olefins, and in particular the selectivity to propylene ($C_3H_6$), is higher for the tin promoted B2 catalyst.

**Claims**

1. Process for preparing olefins from synthesis gas, wherein a catalyst which contains cobalt and manganese is reduced at a temperature higher than 350 °C, and the reduced catalyst is contacted with synthesis gas to effect reaction of the synthesis gas into olefins.

2. Process according to claim 1, wherein before the catalyst is contacted with synthesis gas, the catalyst is subjected to a gas stream comprising a reducing gas consisting of hydrogen.

3. Process according to claim 2, wherein the gas stream comprising a reducing gas consists of from 1 to 99 vol.% of hydrogen, the remainder of the stream comprising an inert gas.

4. Process according to any one of the preceding claims, wherein the reaction temperature is equal to or lower than the reduction temperature.

5. Process according to any one of the preceding claims, wherein the catalyst additionally contains a third element selected from the group consisting of aluminium, gallium, indium, thallium, tin, lead and bismuth.

6. Process according to claim 5, wherein the catalyst contains cobalt, manganese and tin.

7. Process for preparing a supported catalyst, wherein the catalyst contains cobalt, manganese and optionally a third element selected from the group consisting of aluminium, gallium, indium, thallium, tin, lead and bismuth, and is prepared by co-precipitation of a manganese salt and a cobalt salt on a support, optionally followed by impregnation with a salt of the third element, and finally reduction at a temperature higher than 350 °C.

8. Process according to claim 7, wherein the catalyst contains cobalt, manganese and tin.

9. Catalyst obtainable by the process of claim 7 or 8.

Europäisches
Patentamt

European
Patent Office

Office européen
des brevets

# EUROPEAN SEARCH REPORT

Application Number

EP 10 16 7922

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2003/119922 A1 (CHAO WENCHUN [US] ET AL) 26 June 2003 (2003-06-26) <br> * page 2, paragraph 19 * <br> * page 2, paragraph 23 - page 3, paragraph 35 * <br> * page 4, paragraph 40 - paragraph 43 * <br> * page 5, paragraph 55; table 1 * | 1-5,7,9 | INV. <br> C07C1/04 <br> C07C11/02 <br> B01J23/889 <br> B01J29/076 <br> B01J29/26 <br> B01J37/03 |
| X <br><br> A | US 4 778 826 A (JEZL JAMES L [US] ET AL) 18 October 1988 (1988-10-18) <br> * column 19, line 65 - column 21, line 20 * | 1-4,7,9 <br><br> 6,8 | |
| X | COPPERTHWAITE RICHARD G ET AL: "CARBON MONOXIDE HYDROGENATION USING MANGANESE OXIDE BASED CATALYSTS: EFFECT OF OPERATING CONDITIONS ON ALKENE SELECTIVITY." INDUSTRIAL AND ENGINEERING CHEMISTRY RESEARCH 1987 MAY, vol. 26, no. 5, May 1987 (1987-05), pages 869-874, XP008124182 <br> * page 869, column 2 - page 871, column 1; table 1 * <br> * figures 3, 5-6 * | 1-4 | |
| X | COLLEY SAUL ET AL: "CARBON MONOXIDE HYDROGENATION USING COBALT MANGANESE OXIDE CATALYSTS: INITIAL CATALYST OPTIMIZATION STUDIES." INDUSTRIAL AND ENGINEERING CHEMISTRY RESEARCH 1988 AUG, vol. 27, no. 8, August 1988 (1988-08), pages 1339-1344, XP002591558 <br> * page 1341, column 2 * | 1-4 | TECHNICAL FIELDS SEARCHED (IPC) <br><br> C07C <br> B01J |

-/--

The present search report has been drawn up for all claims

| Place of search | Date of completion of the search | Examiner |
|---|---|---|
| The Hague | 5 August 2010 | Patteux, Claudine |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

 

& : member of the same patent family, corresponding
document

EPO FORM 1503 03.82 (P04C01)

Europäisches
Patentamt

European
Patent Office

Office européen
des brevets

# EUROPEAN SEARCH REPORT

Application Number

EP 10 16 7922

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KEYSER M J ET AL: "Fischer-Tropsch studies with cobalt-manganese oxide catalysts: Synthesis performance in a fixed bed reactor" APPLIED CATALYSIS A: GENERAL, ELSEVIER SCIENCE, AMSTERDAM, NL LNKD- DOI:10.1016/S0926-860X(98)00083-0, vol. 171, no. 1, 29 June 1998 (1998-06-29) , pages 99-107, XP004271472 ISSN: 0926-860X * page 101, last paragraph - page 102, paragraph first * ----- | 1-4 | |
| X | HUTCHINGS G.J, COPPERTHWAITE R. G., VAN DER RIET M.: "Low methane selectivity using Co/MnO catalysts for the Fischer-Tropsch reaction : effect of increasing pressure and co-feeding ethene" TOPICS IN CATALYSIS, vol. 2, 1 January 1995 (1995-01-01), pages 163-172, XP002595152 * page 164, paragraph 2.1 - page 165, paragraph 2.2 * * page 165; table 1 * ----- | 1-4 | TECHNICAL FIELDS SEARCHED (IPC) |
| X | VAN DER RIET M., HUTCHINGS G.J., COPERTHWAITE R.G.: "Selective formation of C3 hydrocarbons from CO+H2 using cobalt/manganese oxide catalysts" JOURNAL OF CHEMICAL SOCIETY, CHEMICAL COMMUNICATIONS, 1 January 1986 (1986-01-01), pages 798-799, XP002595153 * page 798, column 2 * * page 799; table 1 * ----- | 1-4 | |

-/--

The present search report has been drawn up for all claims

| Place of search | Date of completion of the search | Examiner |
|---|---|---|
| The Hague | 5 August 2010 | Patteux, Claudine |

EPO FORM 1503 03.82 (P04C01)

Europäisches
Patentamt

European
Patent Office

Office européen
des brevets

**EUROPEAN SEARCH REPORT**

Application Number

EP 10 16 7922

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE 101 57 177 A1 (KATALEUNA GMBH CATALYSTS [DE]) 18 June 2003 (2003-06-18) * example 3 * | 7,9 | |
| X | US 3 956 189 A (WARSHAW ABE ET AL) 11 May 1976 (1976-05-11) * example 1 * | 7,9 | |
| A | EP 1 331 033 A1 (KATALEUNA GMBH CATALYSTS [DE] SHELL INT RESEARCH [NL]) 30 July 2003 (2003-07-30) * paragraph [0051] - paragraph [0055] * | 7,9 | |
| A | EP 0 359 412 A2 (TI CORPORATE SERVICES [GB]) 21 March 1990 (1990-03-21) * page 3, line 30 - line 35 * | 7,8 | |

TECHNICAL FIELDS
SEARCHED      (IPC)

The present search report has been drawn up for all claims

| Place of search | Date of completion of the search | Examiner |
|---|---|---|
| The Hague | 5 August 2010 | Patteux, Claudine |

EPO FORM 1503 03.82 (P04C01)

**ANNEX TO THE EUROPEAN SEARCH REPORT**
**ON EUROPEAN PATENT APPLICATION NO.**

EP 10 16 7922

This annex lists the patent family members relating to the patent documents cited in the above-mentioned European search report.
The members are as contained in the European Patent Office EDP file on
The European Patent Office is in no way liable for these particulars which are merely given for the purpose of information.

05-08-2010

| Patent document cited in search report | | Publication date | Patent family member(s) | | Publication date |
|---|---|---|---|---|---|
| US 2003119922 | A1 | 26-06-2003 | NONE | | |
| US 4778826 | A | 18-10-1988 | NONE | | |
| DE 10157177 | A1 | 18-06-2003 | NONE | | |
| US 3956189 | A | 11-05-1976 | NONE | | |
| EP 1331033 | A1 | 30-07-2003 | AT | 356663 T | 15-04-2007 |
| | | | CA | 2416362 A1 | 22-07-2003 |
| | | | CN | 1433837 A | 06-08-2003 |
| | | | DE | 10202127 A1 | 31-07-2003 |
| | | | DK | 1331033 T3 | 29-05-2007 |
| | | | ES | 2280631 T3 | 16-09-2007 |
| | | | JP | 4253193 B2 | 08-04-2009 |
| | | | JP | 2003260359 A | 16-09-2003 |
| | | | NO | 20030181 A | 23-07-2003 |
| | | | PL | 358053 A1 | 28-07-2003 |
| | | | RU | 2317853 C2 | 27-02-2008 |
| | | | US | 2004220436 A1 | 04-11-2004 |
| | | | US | 2007117714 A1 | 24-05-2007 |
| EP 0359412 | A2 | 21-03-1990 | AU | 4192489 A | 23-03-1990 |
| | | | GB | 2221852 A | 21-02-1990 |
| | | | WO | 9001990 A1 | 08-03-1990 |
| | | | YU | 161789 A1 | 31-12-1990 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **VAN DER LAAN et al.** *Catal. Rev.-Sci. Eng.,* 1999, vol. 41, 255 ff
- **MIRZAEI et al.** *Applied Catalysis A: General,* 2006, vol. 306, 98 ff

- **WEISSERMEL et al.** Industrial Organic Chemistry. Wiley-VCH, 2003, 15-24